# EUROPEAN PATENT APPLICATION

(11) **EP 4 134 102 A1**
(43) Date of publication of application: **15.02.2023**
(21) Application number: 21784091.7
(22) Date of filing: 24.03.2021
(51) Int. Cl.: A61K 47/32, A61K 9/20, A61K 31/522, A61K 31/525, A61K 47/38, A61P 11/06

(54) **SUSTAINED RELEASE BASE**

(30) Priority: 10.04.2020 JP 2020071297
(71) Applicant: Japan Vam & Poval Co., Ltd., Sakai-shi, Osaka 592-8331 (JP)
(72) Inventor: KAWADA Shotaro, Sakai-shi, Osaka 592-8331 (JP); KAWANISHI Masatoshi, Sakai-shi, Osaka 592-8331 (JP)
(74) Representative: Lederer & Keller Patentanwälte Partnerschaft mbB
(86) International application number: PCT/JP2021/012433
(87) International publication number: WO 2021/205887

(57) **Abstract**

A novel sustained-release base is provided. The sustained-release base is a combination of a polyvinyl alcohol-based polymer and a low-substituted hydroxypropyl cellulose.

## Description

### TECHNICAL FIELD

The present invention relates to a sustained-release base and others.

### BACKGROUND ART

A sustained-release preparation is a pharmaceutical preparation in which a medicinal substance is gradually released in the body over a long period of time after administration. This preparation can be administered less frequently than a fast-release preparation. Due to this advantage, sustained-release preparations can reduce the burden on patients and improve patients' compliance and are widely used domestically and internationally.

Various polymers are used as bases in sustained-release preparations, and polyvinyl alcohol (hereinafter also referred to as PVA) is also occasionally used for this purpose.

For example, Patent Literature 1 and 2 disclose that direct compression of a mixture of PVA or a PVA derivative and microcrystalline cellulose (hereinafter also referred to as MCC) provides pharmaceutical preparations with an excellent formability and sustained-release property.

### CITATION LIST

### Patent Literature

Patent Literature 1: Japanese Patent No. 6629835
Patent Literature 2: JP-A2013-241341

### SUMMARY OF INVENTION

### TECHNICAL PROBLEM

An object of the present invention is to provide a novel sustained-release base.

Another object of the present invention is to provide a novel preparation.

Yet another object of the present invention is to provide a method for producing a novel preparation.

### SOLUTION TO PROBLEM

The pharmaceutical preparations described in Patent Literature 1 and 2 have an improved formability and sustained-release property, but their efficacy is not always sufficient.

After intensive studies to achieve the above-mentioned objects, the present inventors found that a combination of PVA and a low-substituted hydroxypropyl cellulose can function as a base in sustained-release preparations. The present inventors further conducted a great deal of examination and then completed the present invention.

That is, the present invention relates to the following.
[1] A sustained-release base comprising a combination of a polyvinyl alcohol-based polymer and a low-substituted hydroxypropyl cellulose.
[2] A solid preparation comprising a polyvinyl alcohol-based polymer and a low-substituted hydroxypropyl cellulose.
[3] The preparation according to the above [2], wherein the preparation is a sustained-release preparation.
[4] The preparation according to the above [2] or [3], wherein the preparation is an oral preparation.
[5] The preparation according to any one of the above [2] to [4], wherein a substance to be formulated into the preparation comprises a medicinal substance.
[6] The base or preparation according to any one of the above [1] to [5], wherein a 4 mass% aqueous solution of the polyvinyl alcohol-based polymer has a viscosity of 15.0 mPa·s or more as measured according to JIS K 6726.
[7] The base or preparation according to any one of the above [1] to [6], wherein the polyvinyl alcohol-based polymer has an average saponification value of 65.0 to 90.0 mol%.
[8] The base or preparation according to any one of the above [1] to [7], wherein the polyvinyl alcohol-based polymer has an average polymerization degree of 1000 or more.
[9] The base or preparation according to any one of the above [1] to [8], wherein the mass ratio of the polyvinyl alcohol-based polymer:the low-substituted hydroxypropyl cellulose is 40:60 to 80:20.
[10] The base or preparation according to any one of the above [1] to [9], wherein the polyvinyl alcohol-based polymer has an average polymerization degree of 1000 or more, and wherein the mass ratio of the polyvinyl alcohol-based polymer:the low-substituted hydroxypropyl cellulose is 40:60 to 80:20.
[11] A method for producing a solid preparation, comprising the step of directly compressing a mixture containing the base according to any one of the above [1] and [6] to [10] and a substance to be formulated into the preparation.
[12] Use of a combination of a polyvinyl alcohol-based polymer and a low-substituted hydroxypropyl cellulose as a sustained-release base.
[13] A method for improving the sustained-release property of a solid preparation, the method comprising using a combination of a polyvinyl alcohol-based polymer and a low-substituted hydroxypropyl cellulose.

### ADVANTAGEOUS EFFECTS OF INVENTION

In one aspect of the present invention, a novel sustained-release base is provided.

In one aspect of the present invention, a sustained-release base that is useful in the production of solid preparations is provided. For example, tableting a mixture containing the sustained-release base of the present invention can efficiently provide a solid preparation (tablet) that is advantageous in terms of hardness and sustained-release property.

With one embodiment of the sustained-release base, a solid preparation having a high hardness is provided. Such a preparation has an excellent formability because it does not readily fall apart after shaping or molding.

In one aspect of the present invention, a novel preparation (particularly, a solid preparation) is provided.

In one aspect of the present invention, a method for producing a novel preparation is provided.

### BRIEF DESCRIPTION OF DRAWINGS

Fig. 1 is a graph showing the time course of the dissolution rate in Examples 1 to 8.
Fig. 2 is a graph showing the time course of the dissolution rate in Reference Examples 1 and 2.

### DESCRIPTION OF EMBODIMENTS

### Sustained-release base

The sustained-release base is a combination of a polyvinyl alcohol-based polymer and a low-substituted hydroxypropyl cellulose.

The sustained-release base of the present invention has only to be a combination of both of these ingredients. The sustained-release base may be a single base that contains both ingredients, or it may be a combination of multiple bases that contains both ingredients as a whole. In other words, a multiple-base system may be a sustained-release base containing a polyvinyl alcohol-based polymer for combination with a low-substituted hydroxypropyl cellulose.

### Polyvinyl alcohol-based polymer

The polyvinyl alcohol-based polymer (may be referred to as a PVA-based polymer, PVA, etc.) may usually be a saponified product of a vinyl ester-based polymer (a polymer at least composed of a vinyl ester as a polymerizable component).

The average saponification value of the PVA-based polymer is, for example, 90.0 mol% or less (e.g., 89.5 mol% or less) and preferably 89.0 mol% or less (e.g., 88.5 mol% or less or 88.0 mol% or less). When the average saponification value of the PVA-based polymer is within this range, the low-substituted hydroxypropyl cellulose seems to be inhibited from exerting disintegrating action, so that the sustained-release effect of the preparation is more likely to be achieved.

For the lower limit, the average saponification value of the PVA-based polymer is, for example, 60.0 mol% or more (e.g., 62.0 mol% or more) and preferably 65.0 mol% or more (e.g., 68.0 mol% or more, 70.0 mol% or more, 75.0 mol% or more, or 80.0 mol% or more).

These upper and lower limits may be combined to set an appropriate range (e.g., 60.0 to 90.0 mol%) for the average saponification value of the PVA-based polymer (the same applies to the others). All combinations of these upper and lower limits are applicable.

The method for measuring the average saponification value of the PVA-based polymer is not particularly limited, and for example, the method for measuring the saponification value specified in JIS K 6726 can be used.

The average polymerization degree of the PVA-based polymer is, for example, 800 or more (e.g., 900 or more, 1000 or more, 1100 or more, 1200 or more, 1300 or more, or 1400 or more), preferably 1500 or more (e.g., 1600 or more), and more preferably 2000 or more (e.g., 2100 or more or 2400 or more). When the average polymerization degree of the PVA-based polymer is within this range, the low-substituted hydroxypropyl cellulose seems to be inhibited from exerting disintegrating action, so that the sustained-release effect of the preparation is more likely to be achieved.

For the upper limit, the average polymerization degree of the PVA-based polymer is, for example, 5000 or less (e.g., 4900 or less), preferably 4500 or less (e.g., 4400 or less), and more preferably 3500 or less (e.g., 3400 or less).

These upper and lower limits may be combined to set an appropriate range (e.g., 1500 to 3500) for the average polymerization degree of the PVA-based polymer (the same applies to the others). All combinations of these upper and lower limits are applicable.

The method for measuring the average polymerization degree of the PVA-based polymer is not particularly limited, and for example, the method for measuring the average polymerization degree specified in JIS K 6726 can be used.

The viscosity of a 4 mass% aqueous solution of the PVA-based polymer (as measured according to JIS K 6726) is, for example, 6.0 mPa·s or more (e.g., 8.0 mPa·s or more), preferably 10.0 mPa·s or more (e.g., 12.0 mPa·s or more), and particularly preferably 15.0 mPa·s or more (e.g., 18.0 mPa·s or more or 20.0 mPa·s or more) from the perspective of the sustained-release property of the preparation.

For the upper limit, the viscosity of a 4 mass% aqueous solution of the PVA-based polymer (as measured according to JIS K 6726) is, for example, 300 mPa·s or less (e.g., 280 mPa·s or less), preferably 240 mPa·s or less (e.g., 220 mPa·s or less), and more preferably 100 mPa·s or less (e.g., 80 mPa·s or less).

These upper and lower limits may be combined to set an appropriate range (e.g., 15 to 240 mPa·s) for the viscosity of a 4 mass% aqueous solution of the PVA-based polymer (the same applies to the others). All combinations of these upper and lower limits are applicable.

A single kind of PVA-based polymer or a combination of two or more kinds of PVA-based polymers may be used.

The PVA-based polymer used may be obtained commercially or synthesized. The method for producing the PVA-based polymer is not particularly limited, and known methods, for example, saponification of a polymer containing a vinyl ester monomer as a polymerizable component (vinyl ester-based polymer), may be used.

The vinyl ester monomer is not particularly limited, and examples include fatty acid vinyl esters [e.g., C₁₋₂₀ fatty acid vinyl esters (e.g., C₁₋₁₆ alkanoic acid vinyl esters) such as vinyl formate, vinyl acetate, vinyl propionate, vinyl butyrate, vinyl caprylate, vinyl versatate, and vinyl monochloroacetate], and aromatic carboxylic acid vinyl esters [e.g., vinyl arenecarboxylates (e.g., C₇₋₁₂ arene carboxylic acid vinyl esters) such as vinyl benzoate].

A single kind of vinyl ester monomer or a combination of two or more kinds of vinyl ester monomers may be used.

The vinyl ester monomer preferably at least comprises a fatty acid vinyl ester (e.g., C₁₋₁₀ alkanoic acid vinyl esters such as vinyl formate, vinyl acetate, vinyl propionate, and vinyl butyrate). In particular, it is industrially preferable that the vinyl ester monomer comprises vinyl acetate.

The vinyl ester-based polymer has a vinyl ester unit, and if necessary, may have an additional monomer unit (a monomer capable of copolymerizing with vinyl ester monomers) (in other words, the vinyl ester-based polymer may be modified with an additional monomer).

The additional monomer is not particularly limited, and examples include, but are not limited to, α-olefins (e.g., ethylene, propylene, etc.), (meth)acrylic acid esters [e.g., (meth)acrylic acid alkyl esters such as methyl (meth)acrylate, ethyl (meth)acrylate, butyl (meth)acrylate, and 2-ethylhexyl (meth)acrylate], unsaturated amides [e.g., (meth)acrylamide, diacetone acrylamide, N-methylolacrylamide, etc.], unsaturated acids {e.g., unsaturated acids [e.g., (meth)acrylic acid, crotonic acid, maleic acid, itaconic acid, fumaric acid, etc.], unsaturated acid esters [unsaturated acid esters other than (meth)acrylic acid esters, e.g., alkyl (methyl, ethyl, propyl, etc.) esters, etc.], unsaturated acid anhydrides (maleic anhydride, etc.), salts of unsaturated acids [e.g., alkali metal salts (e.g., sodium salts, potassium salts, etc.), ammonium salts, etc.], etc.}, glycidyl group-containing monomers [e.g., allyl glycidyl ethers, glycidyl (meth)acrylate, etc.], sulfonic group-containing monomers (e.g., 2-acrylamide-2-methylpropane sulfonic acid, salts thereof, etc.), phosphate group-containing monomers [e.g., acid phosphoxy ethyl (meth)acrylate, acid phosphoxy propyl (meth)acrylate, etc.], vinyl ethers (e.g., alkyl vinyl ethers), allyl alcohols, etc.

A single kind of additional monomer or a combination of two or more kinds of additional monomers may be used.

In the case where the additional monomer is contained as a polymerizable component in the vinyl ester-based polymer, the amount of the additional monomer in the polymerizable components may be, for example, 50 mass% or less, 30 mass% or less, 20 mass% or less, or 10 mass% or less.

The amount of the vinyl ester monomer in the polymerizable components is, for example, 50 mass% or more, preferably 70 mass% or more, more preferably 90 mass% or more and may be 100 mass%.

In the PVA-based polymer, some vinyl alcohol units may be modified by a reaction, such as acetalization, etherification, acetoacetylization, or cationization.

The method for polymerizing the polymerizable components (e.g., the polymerizable components including the vinyl ester monomer such as vinyl acetate) is not particularly limited, and examples include known polymerization methods such as block polymerization, solution polymerization, suspension polymerization, and emulsion polymerization. Among them, solution polymerization using methanol as a solvent is industrially preferred. In the solution polymerization, known initiators such as peroxide initiators and azo initiators can be used, and the polymerization degree of the vinyl ester-based polymer can be adjusted by varying the feed ratio of the polymerizable components and methanol and the polymerization yield. For the production of the PVA-based polymer, commercial vinyl ester-based polymers (such as polyvinyl acetate resin) can be used as a starting material.

The method for saponifying the vinyl ester-based polymer (e.g., polyvinyl acetate) can be a conventional saponification method using an alkaline or acid catalyst. In particular, industrially preferred is alcoholysis, which is performed by adding an alkali such as sodium hydroxide to a solution of the vinyl ester-based polymer (e.g., polyvinyl acetate) in methanol or in a mixed solvent of methanol, water, methyl acetate, etc. and stirring the mixture for cleavage of the acyl group (e.g., acetyl group) of the vinyl ester-based polymer (e.g., polyvinyl acetate).

After that, the obtained mass product, gelled product, or granular product is pulverized, and if needed, the alkali is neutralized; then the solid is separated from the liquid and dried to yield a PVA-based polymer.

For the low-substituted hydroxypropyl cellulose, the degree of substitution (degree of etherification) is, for example, about 0.05 to 1.0 (e.g., 0.07 to 0.8) and preferably about 0.1 to 0.6 (e.g., 0.15 to 0.5).

The degree of substitution may be the average number of substituted hydroxyl groups per glucose unit in the low-substituted hydroxypropyl cellulose.

In the low-substituted hydroxypropyl cellulose, the substitution (mass%) of hydroxypropoxy groups for hydroxyl groups may be, for example, 5 to 16 mass%.

A single kind of low-substituted hydroxypropyl cellulose or a combination of two or more kinds of low-substituted hydroxypropyl celluloses may be used.

The mass ratio of the polyvinyl alcohol-based polymer:the low-substituted hydroxypropyl cellulose in the sustained-release base of the present invention is, for example, about 30:70 to 90:10 (e.g., 33:67 to 87:13), preferably about 35:65 to 85:15 (e.g., 40:60 to 80:20), and more preferably about 43:57 to 78:22 (e.g., 45:55 to 75:25) from the perspective of the sustained-release property and formability of the preparation.

The substance to be mixed with the sustained-release base of the present invention is not particularly limited and may be, for example, a pharmaceutical product, a quasi-pharmaceutical product, a food product, an agrochemical product, or the like. The substance to be mixed with the sustained-release base of the present invention may be an active ingredient, a nutrient (or nutritional ingredient), or the like, and may be a medicinal substance (such as an active pharmaceutical ingredient).

The substance to be mixed with the sustained-release base of the present invention may be an organic substance or an inorganic substance, a mixture thereof, or an organic-inorganic hybrid material.

The medicinal substance or the active ingredient (or the medicinal substance or the active ingredient contained in the substance to be mixed with the sustained-release base of the present invention) is not particularly limited. Examples of the medicinal substance include central nervous system drugs, cerebral metabolism improving agents, cerebral circulation improving agents, antiepileptics, sympathomimetics, cardiovascular drugs, respiratory drugs, gastrointestinal drugs, antibiotics, antitussives and expectorants, antiallergics, antihistamines, dental and stomatological preparations, cardiotonics, antiarrhythmics, diuretics, antipyretic, analgesic and anti-inflammatory agents, autonomic drugs, antidepressants, antipsychotics, anxiolytics, sedative-hypnotics, vasodilators, antihypertensives, vasoconstrictors, peripheral vasodilators, anticoagulants, lipid-lowering agents, choleretics, antimalarial agents, antidiarrheals, psychotropics, chemotherapeutics, diabetes drugs, osteoporosis drugs, skeletal muscle relaxants, analeptics, antispasmodics, antirheumatic drugs, hormone preparations, alkaloid narcotics, sulfa drugs, anti-gout preparations, and antineoplastic agents.

Examples of the central nervous system drug include diazepam, idebenone, aspirin, ibuprofen, paracetamol, naproxen, piroxicam, indomethacin, sulindac, lorazepam, nitrazepam, phenytoin, acetaminophen, ethenzamide, ketoprofen, chlordiazepoxide, and citicoline.

Examples of the cerebral metabolism improving agent include meclofenoxate hydrochloride.

Examples of the cerebral circulation improving agent include vinpocetine.

Examples of the antiepileptic include phenytoin, carbamazepine, and sodium valproate.

Examples of the sympathomimetic include isoproterenol hydrochloride.

Examples of the cardiovascular drug include molsidomine, vinpocetine, propranolol, methyldopa, dipyridamole, furosemide, triamterene, nifedipine, atenolol, spironolactone, metoprolol, pindolol, captopril, isosorbide dinitrate, delapril hydrochloride, meclofenoxate hydrochloride, diltiazem hydrochloride, etilefrine hydrochloride, digitoxin, propranolol hydrochloride, and alprenolol hydrochloride.

Examples of the respiratory drug include antitussives and expectorants, respiratory stimulants, and bronchodilators.

Examples of the antitussive and expectorant include noscapine hydrochloride, carbetapentane citrate, dextromethorphan, dextromethorphan hydrobromide, isoaminile citrate, dimemorfan phosphate, cloperastine hydrochloride, dextromethorphan hydrobromide, theophylline, potassium guaiacolsulfonate, guaifenesin, guaifenesin-codeine phosphate, and ambroxol hydrochloride.

Examples of the respiratory stimulant include levallorphan tartrate.

Examples of the bronchodilator include theophylline, salbutamol, and salbutamol sulfate.

Examples of the gastrointestinal drug include gastrointestinal agents, antacids, antiulcer agents, digestive enzyme preparations, laxatives, and antiemetics.

Examples of the gastrointestinal agent include stomachic digestive agents (e.g., diastase, saccharated pepsin, scopolia extract, cellulase AP3, lipase AP, and cinnamon oil) and intestinal regulators (e.g., berberine chloride, antibiotics-resistant lactic acid bacteria, and bifidobacteria).

Examples of the antacid include magnesium carbonate, sodium hydrogen carbonate, magnesium aluminometasilicate, synthetic hydrotalcite, precipitated calcium carbonate, and magnesium oxide.

Examples of the antiulcer agent include 5-aminosalicylic acid, lansoprazole, omeprazole, rabeprazole, cimetidine, famotidine, ranitidine, ranitidine hydrochloride, pirenzepine hydrochloride, and antiulcer benzimidazole drugs such as 2-[[3-methyl-4-(2,2,2-trifluoroethoxy)-2-pyridyl]methylsulfinyl]benzimidazole and 5-methoxy-2-[(4-methoxy-3,5-dimethyl-2-pyridyl)methylsulfinyl]benzimidazole.

Examples of the digestive enzyme preparation include pancreatin.

Examples of the laxative include bisacodyl.

Examples of the antiemetic include difenidol hydrochloride and metoclopramide.

Examples of the antibiotic include cephems such as cephalexin, amoxicillin, cefaclor, pivmecillinam hydrochloride, cefotiam hexetil hydrochloride, cefadroxil, cefixime, cefditoren pivoxil, cefteram pivoxil and cefpodoxime proxetil; synthetic antibacterial agents such as ampicillin, ciclacillin, nalidixic acid, and enoxacin; monobactams such as carumonam sodium; macrolides such as erythromycin; penems; and carbapenems.

Examples of the antiallergic include amlexanox and seratrodast.

Examples of the antihistamine include diphenhydramine hydrochloride, promethazine, promethazine hydrochloride, isothipendyl hydrochloride, and dl-chlorpheniramine maleate.

Examples of the dental and stomatological preparation include oxytetracycline, triamcinolone acetonide, chlorhexidine hydrochloride, and lidocaine.

Examples of the cardiotonic include digoxin and caffeine.

Examples of the arrhythmic include procainamide hydrochloride, propranolol hydrochloride, and pindolol.

Examples of the diuretic include caffeine, furosemide, isosorbide, and hydrochlorothiazide.

Examples of the antipyretic, analgesic and anti-inflammatory agent include acetaminophen, aspirin, ibuprofen, ethenzamide, diphenhydramine hydrochloride, dl-chlorpheniramine maleate, diclofenac sodium, dihydrocodeine phosphate, salicylamide, aminopyrine, noscapine, methylephedrine hydrochloride, phenylpropanolamine hydrochloride, serrapeptase, lysozyme chloride, tolfenamic acid, mefenamic acid, flufenamic acid, ketoprofen, indomethacin, bucolome, pentazocine, caffeine, anhydrous caffeine, sulpyrine, hydromorphone hydrochloride, and tapentadol hydrochloride.

Examples of the autonomic agent include dihydrocodeine phosphate and dl-methylephedrine hydrochloride, atropine sulfate, acetylcholine chloride, and neostigmine.

Examples of the vasodilator include nifedipine, carbocromen hydrochloride, molsidomine, and verapamil hydrochloride.

Examples of the antihypertensive include captopril, delapril hydrochloride, hydralazine hydrochloride, labetalol hydrochloride, manidipine hydrochloride, candesartan cilexetil, methyldopa, and perindopril erbumine.

Examples of the vasoconstrictor include phenylephrine hydrochloride.

Examples of the peripheral vasodilator include cinnarizine.

Examples of the anticoagulant include dicoumarol.

Examples of the lipid-lowering agent include cerivastatin sodium, simvastatin, pravastatin sodium, and atorvastatin calcium hydrate.

Examples of the choleretic include dehydrocholic acid and trepibutone.

Examples of the antimalarial agent include quinine hydrochloride.

Examples of the antidiarrheal include loperamide hydrochloride.

Examples of the psychotropic include chlorpromazine and reserpine.

Examples of the antidepressant include amphetamine, imipramine, maprotiline hydrochloride, and paroxetine hydrochloride.

Examples of the antipsychotic include paliperidone.

Examples of the anxiolytic include diazepam, alprazolam, and chlordiazepoxide.

Examples of the sedative-hypnotic include estazolam, diazepam, nitrazepam, perlapine, and phenobarbital sodium.

Examples of the chemotherapeutic include sulfamethizole.

Examples of the diabetes drug include glymidine sodium, glipizide, phenformin hydrochloride, buformin hydrochloride, metformin, metformin hydrochloride, tolbutamide, voglibose, pioglitazone hydrochloride, glibenclamide, and troglitazone.

Examples of the osteoporosis drug include ipriflavone.

Examples of the skeletal muscle relaxant include methocarbamol.

Examples of the analeptic include vitamins and their derivatives [e.g., vitamin A, vitamin B 1, fursultiamine, vitamin B2 (riboflavin), vitamin B6, vitamin B12, vitamin C, vitamin D, vitamin E, vitamin K, calcium pantothenate, tranexamic acid, etc.], minerals (e.g., calcium, magnesium, iron, etc.), proteins, amino acids, oligosaccharides, and herbal medicines.

Examples of the antispasmodic include meclizine hydrochloride, dimenhydrinate, scopolamine hydrobromide, diphenhydramine hydrochloride, and papaverine hydrochloride.

Examples of the antirheumatic drug include methotrexate and bucillamine.

Examples of the hormone preparation include liothyronine sodium, dexamethasone sodium phosphate, prednisolone, oxendolone, and leuprorelin acetate.

Examples of the alkaloid narcotic include opium, morphine hydrochloride, ipecac, oxycodone hydrochloride, opium alkaloids hydrochloride, and cocaine hydrochloride.

Examples of the sulfa drug include sulfisomidine and sulfamethizole.

Examples of the anti-gout preparation include allopurinol and colchicine.

Examples of the antineoplastic agent include 5-fluorouracil, uracil, and mitomycin.

The substance to be mixed with the sustained-release base of the present invention may further contain an additional ingredient (an additive usually used in this field, for example, a filler, a disintegrant, a lubricant, an antiagglomerant, a solubilizer, etc.).

Examples of the filler include saccharides, such as sucrose, lactose, mannitol, and glucose, starch, crystalline cellulose, calcium phosphate, calcium sulfate, etc. Preferred are lactose, mannitol, crystalline cellulose, etc.

Examples of the disintegrant include carmellose or a salt thereof, croscarmellose sodium, sodium carboxymethyl starch, crospovidone, microcrystalline cellulose, microcrystalline cellulose-carmellose sodium, etc.

Examples of the lubricant or the antiagglomerant include talc, magnesium stearate, calcium stearate, colloidal silica, stearic acid, waxes, hydrogenated oils, polyethylene glycols, sodium benzoate, etc.

Examples of the solubilizer include organic acids, such as fumaric acid, succinic acid, malic acid, and adipic acid, etc.

One of these additives or two or more of them may be used. The amount of the additional ingredient (the additive) can be determined as appropriate according to, for example, the type of the drug.

A single kind of substance to be mixed with the sustained-release base of the present invention or a combination of two or more kinds of substances to be mixed with said base may be used.

The substance to be mixed with the sustained-release base of the present invention may be a solid at ordinary temperature (e.g., 10 to 40°C).

The substance to be mixed with the sustained-release base of the present invention is preferably in the form of powder (or powder particles). The particle size of the powder (active ingredient, active ingredient powder, etc.) is not particularly limited, and the average particle diameter is, for example, about 500 µm or less (e.g., 5 to 400 µm), preferably about 300 µm or less, and more preferably about 100 µm or less (e.g., 10 to 80 µm).

The average particle diameter of the powder may be measured with a laser-type particle size distribution measuring instrument, etc.

### Solid preparation, etc.

The present invention also encompasses a solid preparation containing a polyvinyl alcohol-based polymer and a low-substituted hydroxypropyl cellulose. The polyvinyl alcohol-based polymer and the low-substituted hydroxypropyl cellulose may be as exemplified above. The substance to be formulated into the preparation of the present invention may be a substance to be mixed with the sustained-release base of the present invention. The substance to be formulated into the preparation is usually in the form of powder particles.

The preparation of the present invention may be, for example, a tablet, granule, or capsule preparation, and is preferably a tablet preparation.

The preparation of the present invention is suitable for use as an oral preparation.

The preparation of the present invention may further contain an additional ingredient besides the polyvinyl alcohol-based polymer and the low-substituted hydroxypropyl cellulose (and the substance to be formulated into the preparation).

The additional ingredient may be an additive usually used in this field (e.g., a disintegrant, a lubricant, an antiagglomerant, a solubilizer, etc.).

Examples of the disintegrant include carmellose or a salt thereof, croscarmellose sodium, sodium carboxymethyl starch, crospovidone, microcrystalline cellulose, microcrystalline cellulose-carmellose sodium, etc.

Examples of the lubricant or the antiagglomerant include talc, magnesium stearate, calcium stearate, colloidal silica, stearic acid, waxes, hydrogenated oils, polyethylene glycols, sodium benzoate, etc.

Examples of the solubilizer include organic acids, such as fumaric acid, succinic acid, malic acid, and adipic acid, etc.

One of these additives or two or more of them may be used.

The amount of the additive can be determined as appropriate according to, for example, the type of the ingredient contained in the preparation.

The amount of the sustained-release base of the present invention in the preparation is, for example, about 40 to 99.5 mass% (e.g., 42 to 99 mass%), preferably about 45 to 98.5 mass% (e.g., 50 to 98 mass%), and more preferably about 52 to 97.5 mass% (e.g., 55 to 97 mass%).

The amount of the polyvinyl alcohol-based polymer in the preparation is, for example, about 20 to 60 mass% (e.g., 22 to 58 mass%), preferably about 25 to 55 mass% (e.g., 27 to 52 mass%), and more preferably about 30 to 50 mass% (e.g., 33 to 48 mass%).

The amount of the low-substituted hydroxypropyl cellulose in the preparation is, for example, about 5 to 60 mass% (e.g., 7 to 58 mass%), preferably about 10 to 55 mass% (e.g., 12 to 52 mass%), and more preferably about 15 to 50 mass% (e.g., 33 to 48 mass%).

The mass ratio of the polyvinyl alcohol-based polymer:the low-substituted hydroxypropyl cellulose in the preparation is, for example, about 30:70 to 90:10 (e.g., 33:67 to 87:13), preferably about 35:65 to 85:15 (e.g., 40:60 to 80:20), and more preferably about 43:57 to 78:22 (e.g., 45:55 to 75:25) from the perspective of the sustained-release property and formability of the preparation.

The shape of the preparation is not particularly limited. In the case where the preparation is a tablet, the shape of the tablet may be a disk shape, a lens shape, a pole shape, or some other shape.

The size of the preparation is not particularly limited. In the case where the preparation is a tablet, the size of the tablet may be, for example, 3 mm or more (e.g., 4 to 15 mm, 5 to 12 mm, 8 to 11 mm, etc.) in diameter (maximum diameter).

Tablets can be produced by tableting a mixture (a powder for tableting) at least containing a polyvinyl alcohol-based polymer, a low-substituted hydroxypropyl cellulose, and a substance to be formulated into the preparation (or at least containing the sustained-release base of the present invention and a substance to be formulated into the preparation).

The powder for tableting may contain an additional ingredient as described above.

The tableting method is not particularly limited, and conventional tableting methods can be employed. Particularly, direct compression (direct tableting) is preferred. For direct compression (direct tableting), a powder for tableting is directly compressed and molded or shaped in a tableting machine.

The tablet may have a coat layer on the surface of the tablet.

The component of the coat layer in the coated tablet is not particularly limited, and examples include resin materials, such as cellulose resin (e.g., hydroxypropylmethyl cellulose, hydroxypropylmethylcellulose acetate succinate, etc.), and polyvinyl alcohol-based polymers.

The preparation of the present invention may be a sustained-release preparation.

For the sustained-release preparation of the present invention, the time taken for the dissolution rate of the active ingredient (e.g., an active ingredient, a nutrient, a medicinal substance, etc.) to reach 80% or more (e.g., 80%) (dissolution time) can vary with the type of the active ingredient, but is, for example, at least 2 hours or more, preferably 3 hours or more (e.g., 4 hours or more, 5 hours or more, 6 hours or more, or 7 hours or more).

For the upper limit, the time taken for the dissolution rate of the active ingredient to reach 80% or more (e.g., 80%) is not particularly limited and is, for example, 20 hours or less, 19 hours or less, 18 hours or less, 17 hours or less, 16 hours or less, or 15 hours or less.

For the sustained-release preparation of the present invention, the time taken for the dissolution rate of the active ingredient to reach 50% can vary with the type of the active ingredient, but is, for example, 20 minutes or more, preferably 30 minutes or more (e.g., 40 minutes or more), more preferably 60 minutes or more (e.g., 80 minutes or more).

For the upper limit, the time taken for the dissolution rate of the active ingredient to reach 50% is not particularly limited and is, for example, 20 hours or less, 19 hours or less, 18 hours or less, 17 hours or less, 16 hours or less, or 15 hours or less.

The method for measuring the dissolution time is not particularly limited and, for example, the Japanese Pharmacopoeia dissolution test method 2 (paddle method) can be used. The Japanese Pharmacopoeia dissolution test method 2 (paddle method) can be performed using 900 mL of distilled water as the test solution at a paddle rotation speed of 50 rpm.

### EXAMPLES

Hereinafter, the present invention will be described in more detail by examples, but the present invention is not limited thereto.

In the following Examples and Comparative Examples, "percentage (%)" and "part" are on a mass basis unless otherwise specified.

The experimental conditions are as follows.

### Average saponification value, average polymerization degree, and viscosity of 4 mass% aqueous solution

These were measured according to JIS K 6726.

### Tableting conditions

Apparatus: Rotary tablet press VIRGO (manufactured by KIKUSUI SEISAKUSHO LTD.)
Tableting pressure: 15 kN
Tablet press rotational speed: 10 rpm
Tablet weight: 200 mg
Tablet diameter: 8 mmϕ

### Evaluation of tablet hardness

The hardness of the obtained tablet was measured using a tablet hardness tester (TBH125 manufactured by ERWEKA). The measurement was performed 6 times, and the average value of the 6 results was used as the measured value.

### Evaluation of dissolution rate

A dissolution test was performed under the test conditions described below. The time taken for the dissolution rate to reach 80% was measured and calculated.
Test method: the Japanese Pharmacopoeia dissolution test method 2 (paddle method)
Test fluid: 900 mL of distilled water
Paddle rotation speed: 50 rpm
Samples: tablets shown in Examples and Comparative Examples
Detection wavelengths: UV 444 nm (for riboflavin), UV 270 nm (for theophylline)

The PVA-based polymers (PVA1 to PVA3) used in the following Examples and Reference Examples are listed in Table 1.

The PVAs with the product names listed in Table 1 are PVA products manufactured by JAPAN VAM & POVAL CO., LTD.

All of the PVA-based polymers were pulverized so that the average particle size would be about 100 µm.

**[Table 1]**

| | Product name | Average polymerization degree | Average saponification value (mol%) | Viscosity of 4 mass% aqueous solution (mPa·s) |
|---|---|---|---|---|
| PVA1 | JP-24 | 2400 | 88.0 | 45.2 |
| PVA2 | JP-18 | 1800 | 88.0 | 25.5 |
| PVA3 | - | 2400 | 65.0 | 47.5 |

The synthesis method of PVA3 is shown below.

### Synthesis Example 1

In a reaction vessel equipped with a stirrer, a condenser, a nitrogen gas inlet, and an initiator inlet, 140 parts of methanol and 860 parts of vinyl acetate monomer were fed. After nitrogen replacement in the system, the temperature was heated to 60°C. When reflux started, 2.0 parts of a 1.5% methanol solution of 2,2'-azobis(2,4-dimethylvaleronitrile) was added as an initiator to initiate polymerization. During the polymerization, the system was maintained at 70°C at normal pressure under nitrogen gas flow. After 15 minutes from the start of polymerization, 2.0 parts of a 2% methanol solution of 2,2'-azobis(2,4-dimethylvaleronitrile) was further added. After 2.3 hours from the start of polymerization, the yield of vinyl acetate in the reaction reached 52%, and the system was cooled to terminate the polymerization. While methanol vapor was added to the reaction mixture, residual vinyl acetate monomer was evaporated off, thus giving a 40% methanol solution of polyvinyl acetate.

Next, 500 parts of the 40% methanol solution of polyvinyl acetate obtained above, 100 parts of methanol, 6 parts of water, and 6.3 parts of a 5.2% methanol solution of sodium hydroxide were mixed well. The mixture was subjected to saponification at 40°C for 40 minutes. The resulting gelled product was pulverized, neutralized, washed with methanol, and dried to give a PVA-based polymer 3 (PVA3) with a saponification value of 65.0 mol% and an average polymerization degree of 2400.

### Example 1

The amounts of ingredients, excluding magnesium stearate (St-Mg), in the listed in Table 2 were placed into a polyethylene bag. The bag was inflated with air and shaken 100 times for mixing the contents. Subsequently, St-Mg was added to the bag, and the bag was inflated with air and shaken 30 times for mixing the contents. The resulting mixture was compressed into tablets. For the obtained tablets, tablet hardness and the dissolution time were measured. The results are shown in Table 2. In Table 2, L-HPC stands for low-substituted hydroxypropyl cellulose. The L-HPC used was NBD-021 (Shin-Etsu Chemical Co., Ltd.).

### Examples 2 to 8

In each Example, tableting was performed in the same manner as described in Example 1, except that the PVA-based polymer and other ingredients listed in Table 2 were used in the amounts described in Table 2. For the obtained tablets, tablet hardness and the dissolution time were measured. The results are shown in Table 2.

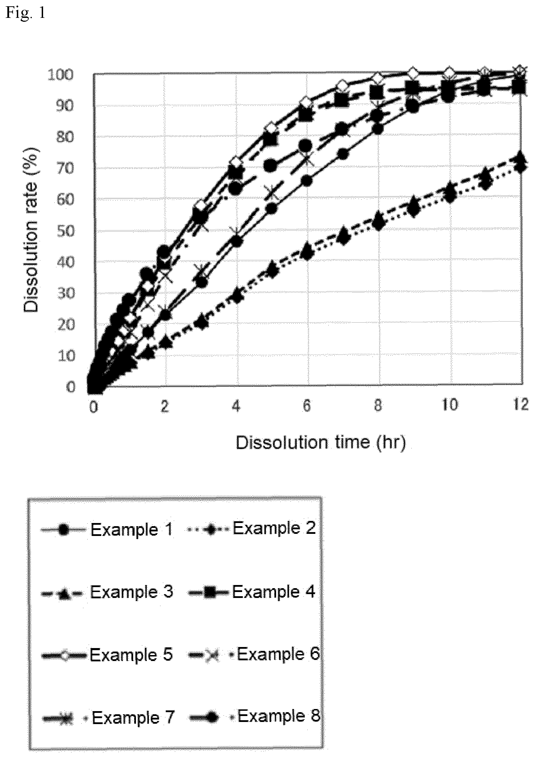

The time course of the dissolution rate in Examples 1 to 8 is shown in Fig. 1.

### Reference Examples 1 and 2

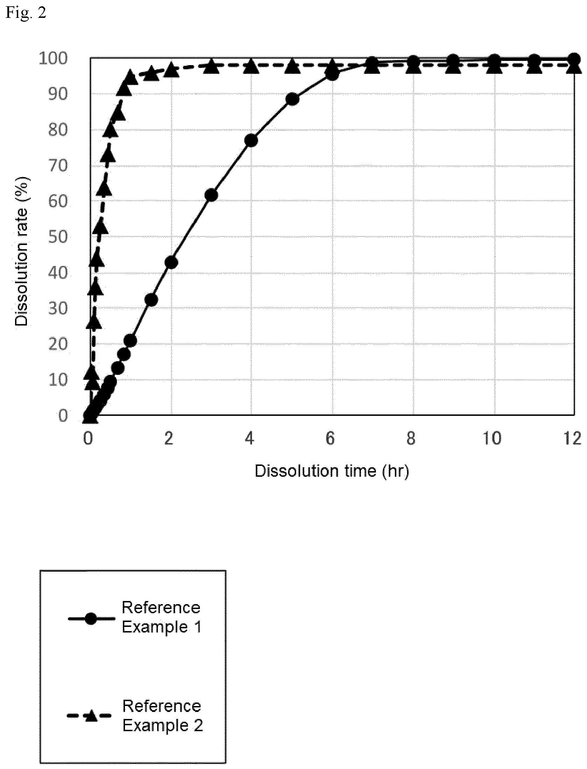

In each Example, tableting was performed in the same manner as described in Example 1, except that the PVA-based polymer and other ingredients listed in Table 2 were used in the amounts described in Table 2. For the obtained tablets, tablet hardness and the dissolution time were measured. The results are shown in Table 2. In Table 2, Cl-PVP stands for cross-linked polyvinylpyrrolidone (Kollidon CL, BASF Japan). The time course of the dissolution rate in Reference Examples 1 and 2 is shown in Fig. 2.

**[Table 2]**

| | PVA | Amount (parts by mass) | | | | | | | | Tablet hardness (N) | Time taken for dissolution rate to reach 80% (hours) |
|---|---|---|---|---|---|---|---|---|---|---|---|
| | | PVA | L-HPC | Cl-PVP | Lactose | Riboflavin | Theophylline | St-Mg | Total | | |
| Example 1 | PVA1 | 40 | 20 | - | 35 | 5 | - | 0.5 | 100.5 | 144 | 7.8 |
| Example 2 | PVA1 | 40 | 40 | - | 15 | 5 | - | 0.5 | 100.5 | 161 | 14.0 |
| Example 3 | PVA1 | 36 | 54 | - | 5 | 5 | - | 0.5 | 100.5 | 182 | 13.5 |
| Example 4 | PVA1 | 40 | 10 | - | 45 | 5 | - | 0.5 | 100.5 | 122 | 5.2 |
| Example 5 | PVA1 | 40 | 5 | - | 50 | 5 | - | 0.5 | 100.5 | 105 | 4.6 |
| Example 6 | PVA2 | 60 | 20 | - | 15 | 5 | - | 0.5 | 100.5 | 138 | 5.2 |
| Example 7 | PVA3 | 40 | 20 | - | 35 | 5 | - | 0.5 | 100.5 | 140 | 6.8 |
| Example 8 | PVA1 | 40 | 20 | - | 0 | - | 40 | 0.5 | 100.5 | 156 | 6.7 |
| Reference Example 1 | PVA1 | 40 | | | 55 | 5 | - | 0.5 | 100.5 | 100 | 4.1 |
| Reference Example 2 | PVA1 | 40 | - | 20 | 35 | 5 | - | 0.5 | 100.5 | 110 | 0.6 |

As shown in the Examples in Table 2, the combined use of the PVA-based polymer and the low-substituted hydroxypropyl cellulose was effective in producing tablets having both a high formability and an excellent sustained-release property.

### INDUSTRIAL APPLICABILITY

The present invention provides a novel sustained-release base. The sustained-release base is useful for various applications including the production of sustained-release preparations.

## Claims

1. A sustained-release base comprising a combination of a polyvinyl alcohol-based polymer and a low-substituted hydroxypropyl cellulose.

2. A solid preparation comprising a polyvinyl alcohol-based polymer and a low-substituted hydroxypropyl cellulose.

3. The preparation according to claim 2, wherein the preparation is a sustained-release preparation.

4. The preparation according to claim 2 or 3, wherein the preparation is an oral preparation.

5. The preparation according to any one of claims 2 to 4, wherein a substance to be formulated into the preparation comprises a medicinal substance.

6. The base or preparation according to any one of claims 1 to 5, wherein a 4 mass% aqueous solution of the polyvinyl alcohol-based polymer has a viscosity of 15.0 mPa·s or more as measured according to JIS K 6726.

7. The base or preparation according to any one of claims 1 to 6, wherein the polyvinyl alcohol-based polymer has an average saponification value of 65.0 to 90.0 mol%.

8. The base or preparation according to any one of claims 1 to 7, wherein the polyvinyl alcohol-based polymer has an average polymerization degree of 1000 or more.

9. The base or preparation according to any one of claims 1 to 8, wherein the mass ratio of the polyvinyl alcohol-based polymer:the low-substituted hydroxypropyl cellulose is 40:60 to 80:20.

10. The base or preparation according to any one of claims 1 to 9, wherein the polyvinyl alcohol-based polymer has an average polymerization degree of 1000 or more, and wherein the mass ratio of the polyvinyl alcohol-based polymer:the low-substituted hydroxypropyl cellulose is 40:60 to 80:20.

11. A method for producing a solid preparation, comprising the step of directly compressing a mixture containing the base according to any one of claims 1 and 6 to 10 and a substance to be formulated into the preparation.
